# EUROPÄISCHE PATENTANMELDUNG

(11) **EP 4 620 409 A2**
(43) Veröffentlichungstag der Anmeldung: **24.09.2025**
(21) Anmeldenummer: 25192876.8
(22) Anmeldetag: 30.07.2025
(51) Int. Cl.: A61B 17/16

(54) **MARKRAUMBOHRERBOHRWELLE UND MARKRAUMBOHRER**

(71) Anmelder: MMN-PW Markraumbohrer GmbH, 24111 Kiel (DE)
(72) Erfinder: WITTE, Peter, 24111 Kiel (DE)
(74) Vertreter: Seemann & Partner Patentanwälte mbB

(57) **Zusammenfassung**

Die Erfindung betrifft eine Markraumbohrerbohrwelle 10 und einen Markraumbohrer 1 mit der Markraumbohrerbohrwelle 10. Die Markraumbohrerbohrwelle 10 umfasst einen längserstreckten Schaft 19. Der Schaft 19 weist einen Wellenkern 18 mit einer durchgehenden, längsaxialen Bohrung 15 auf. Der Wellenkern 18 ist aus Nitinol gefertigt. An einem ersten Ende 11 des Schafts 19 ist ein Bohreradapter 13 unlösbar und gasdicht mit dem Wellenkern 18 verbunden, wobei an einem gegenüberliegenden zweiten Ende 12 ein Antriebsadapter 14 unlösbar und gasdicht mit dem Wellenkern 18 verbunden ist. Eine Mantelfläche 16 des Wellenkerns 18 ist von einer impulsverringernden Hülse 17 aus Kunststoff umschlossen. Die Hülse 17 erstreckt sich von dem ersten Ende 11 durchgehend bis zu dem zweiten Ende 12.

## Beschreibung

Die Erfindung betrifft eine Markraumbohrerbohrwelle. Zudem betrifft die Erfindung einen Markraumbohrer.

Markraumbohrer werden vielfach genutzt, um die Markhöhle im Knochen eines Patienten aufzubohren, damit Implantate eingebracht werden können. Um zu dem aufzubohrenden Bereich zu gelangen, sind die Bohrwellen solcher Markraumbohrer in der Regel flexibel ausgebildet. Die flexiblen Elemente solcher Bohrwellen sind jedoch zumeist anfällig für Keimbelastungen. Aus diesem Grund ist es notwendig, den gesamten Markraumbohrer nach jeder Anwendung gründlich aufzubereiten und zu sterilisieren. Gerade im Bereich der flexiblen Bohrwelle ist die notwendige Aufbereitung und Sterilisierung jedoch nicht immer im erforderlichen Maße möglich, da sich in den Zwischenräumen zwischen den flexiblen Elementen schwer zu entfernende Keime ansetzen können.

Eine Möglichkeit, diesem Problem zu entgehen, besteht darin, den Markraumbohrer als Single Use Bauteil auszubilden und nach jeder Anwendung zu ersetzen. Dies führt jedoch zu hohen Kosten und einem hohen Material- und Fertigungsaufwand.

In dem Dokument "Bohrwelle für einen Markraumbohrer, Markraumbohrer, Impulsverringernde Beschichtung, Verwendung einer Beschichtung aus Polyetheretherketon auf einem aus Nitinol gefertigten Schaft einer Bohrwelle eines Markraumbohrers und Verfahren zum Wiederaufbereiten eines Markraumbohrers", welches am 24 Juli 2024 auf Questel Research Disclosure veröffentlicht wurde, ist eine Markraumbohrerwelle aus Nitinol gezeigt, welche mit Polyetheretherketon beschichtet ist. Auf diese Weise wird verhindert, dass bei einem Bruch des Nitinolschafts Splitter ins Körperinnere gelangen. Allerdings ist die Herstellung einer solchen Bohrwelle schwierig, da zum Auftragen der Beschichtung eine Temperatur von etwa 400°C notwendig ist. Da es sich bei Nitinol um eine Formgedächtnislegierung handelt, deren Transformationstemperatur deutlich unter dieser Temperatur liegt, wird die Herstellung einer solchen Bohrwelle erschwert.

Die Aufgabe der Erfindung besteht darin, bei der Nutzung eines Markraumbohrers eine gute Aufbereitung und Sterilisierung sicherzustellen, ohne einen zu großen Materialeinsatz und Fertigungsaufwand zu benötigen.

Diese Aufgabe wird gelöst durch eine Markraumbohrerbohrwelle, umfassend einen längserstreckten Schaft, wobei der Schaft einen Wellenkern mit einer durchgehenden, längsaxialen Bohrung aufweist, wobei der Wellenkern aus Nitinol gefertigt ist, wobei an einem ersten Ende des Schafts ein Bohreradapter unlösbar mit dem Wellenkern verbunden ist, wobei an einem gegenüberliegenden zweiten Ende ein Antriebsadapter unlösbar mit dem Wellenkern verbunden ist, wobei eine Mantelfläche des Wellenkerns von einer impulsverringernden Hülse aus Kunststoff umschlossen ist, wobei sich die Hülse von dem ersten Ende durchgehend bis zu dem zweiten Ende erstreckt.

Eine Bohrwelle aus Nitinol weist die erforderlichen Materialeigenschaften auf, unter anderem die notwendige Biegsamkeit und Torsionsfähigkeit, damit die Bohrwelle ohne zusätzliche flexible Elemente auskommt. Der Wellenkern, also der Schaft ohne die Hülse, besteht insbesondere vollständig aus Nitinol. Jedoch hat Nitinol den Nachteil, dass im Falle eines Bruchs der Markraumbohrerbohrwelle Splitter entstehen, die in den Körperinnenraum des Patienten gelangen würden und dort großen Schaden anrichten könnten. Um dies zu verhindert, ist eine Hülse aus Kunststoff um das Nitinolrohr vorgesehen. Insbesondere handelt es sich bei dem Kunststoff um Polyetheretherketon (PEEK), Polyphenylensulfon (PPSU) oder Polypropylen (PP). Beispielsweise ist die Hülse aus PEEK gefertigt. Durch eine Hülse aus Kunststoff werden die Splitter des Nitinolschafts im Falle eines Bruchs daran gehindert, in den Körperinnenraum des Patienten einzudringen. Die Hülse wirkt somit als impulsverringerndes Element bzw. impulsverringernder Mantel.

Insbesondere ist an dem ersten Ende des Schafts der Bohreradapter unlösbar und gasdicht mit dem Wellenkern verbunden und/oder an dem zweiten Ende der Antriebsadapter unlösbar und gasdicht mit dem Wellenkern verbunden. Durch eine gasdichte Verbindung lässt sich die Markraumbohrerbohrwelle besser autoklavieren.

Der Wellenkern weist in der Mitte die Bohrung auf. Diese Bohrung unterscheidet Markraumbohrerbohrwellen von vielen anderen Arten von Bohrwellen. Durch die Bohrung wird die Stabilität im Vergleich zu einer Bohrwelle ohne Bohrung verringert, wodurch die impulsverringernde Hülse notwendig wird.

Im Gegensatz zu einer -Beschichtung aus Kunststoff, beispielsweise eine PEEK Beschichtung, die direkt auf die Mantelfläche aufgebracht ist, handelt es sich bei der Hülse um ein separates Bauteil, das über den Wellenkern geschoben wird. Dies erleichtert die Montage und Herstellung der Markraumbohrerbohrwelle deutlich, da die Hülse und der Wellenkern separat hergestellt und erst später zusammengesetzt werden. Dadurch wird vermieden, dass der Wellenkern aus Nitinol während der Fertigung über die Transformationstemperatur aufgeheizt werden muss.

Insbesondere besteht zwischen der Hülse und der Mantelfläche des Wellenkerns ein Spaltmaß. Es hat sich herausgestellt, dass durch das Spaltmaß der Impuls von Nitinolsplittern noch stärker reduziert wird, als wenn eine Beschichtung der gleichen Dicke direkt auf die Mantelfläche aufgetragen wird. Eine Markraumbohrerbohrwelle ohne Spaltmaß lässt sich beispielsweise durch aufschrumpfen der Hülse auf den Wellenkern fertigen und ist eine weitere Ausführungsform.

Insbesondere ist der Bohradapter und/oder der Antriebsadapter an dem Wellenkern gasdicht angeschweißt. Die Hülse ist insbesondere in den Bohradapter und/oder der Antriebsadapter eingesteckt. Somit lässt sich die Hülse nach dem Durchführen der Schweißung nicht mehr ablösen, so dass der Wellenkern, die Hülse, der Bohradapter und der Antriebsadapter ein einzelnes Bauteil bilden. Dieses Bauteil ist insbesondere als Ganzes aufbereitbar und/oder autoklavierbar.

Der Bohradapter und/oder der Antriebsadapter ist insbesondere aus einem nicht splitternden Material gefertigt, insbesondere Stahl.

Vorzugsweise ist die Mantelfläche wenigstens von dem Bohreradapter bis zum Antriebsadapter durchgehend von der Hülse ummantelt. Mit anderen Worten ist die gesamte Mantelfläche von dem ersten Ende, an dem der Bohreradapter fixiert ist, bis zum zweiten Ende, an dem der Antriebsadapter fixiert ist, vollständig und vollflächig umhüllt mit der Hülse. Durch eine durchgehende Ummantelung der Mantelfläche wird ein Eindringen von Splittern in den Patientenkörper zuverlässig unterbunden. Selbst eine kleine Unterbrechung oder Aussparung in der Hülse zwischen den Adaptern könnte dazu führen, dass Splitter ungehindert in den Patientenkörper gelangen und zu inneren Verletzungen führen. Lediglich die äußeren Endabschnitte des Wellenkerns, die innerhalb der Adapter angeordnet sind, sind insbesondere so ausgebildet, dass sie nicht von der Hülse umhüllt sind. Mit anderen Worten ist der Wellenkern länger als die Hülse, so dass die Endabschnitte über die Hülse herausragen. Für diese Endabschnitte sorgen die Adapter für einen Splitterschutz. Zudem ermöglicht ein nicht umhüllter Endabschnitt eine feste Fixierung, beispielsweise Verschweißung, des Wellenkerns mit den Adaptern.

Bevorzugt weist die Hülse eine Wandungsstärke von wenigstens 0,3 mm, insbesondere wenigstens 0,5 mm, auf. Beispielsweise hat die ummantelnde Hülse eine Dicke von etwa 0,5 mm oder etwa 1 mm. Eine solche Wandungsstärke ist ausreichend, um ein Eindringen der Nitinolsplitter in den Patientenkörper zu verhindern, ohne dass die Flexibilität der Bohrwelle unnötig eingeschränkt wird.

Vorzugsweise weist die Hülse ein Spaltmaß von wenigstens 0,01 mm zur Mantelfläche des Wellenkerns auf. Insbesondere beträgt das Spaltmaß 0,01 mm bis 0,02 mm. Mit anderen Worten ist ein Innenradius der Hülse wenigstens 0,01 mm, insbesondere 0,01 mm bis 0,02 mm, größer als ein Außenradius des Wellenkerns. Ein solches Spaltmaß hat sich als sinnvoll erwiesen um eine gute Halterung an der Markraumbohrerbohrwelle zu erreichen und gleichzeitig die nötigen impulsverringernden Eigenschaften bereitzustellen.

Gemäß einer anderen Ausführungsform ist die Hülse auf den Wellenkern aufgeschrumpft, so dass die Hülse insbesondere ein Spaltmaß von weniger als 0,01 mm zur Mantelfläche des Wellenkerns aufweist. Das Aufschrumpfen stellt eine andere Art der Herstellung der Markraumbohrerbohrwelle dar, bei der lediglich ein minimales Spaltmaß zwischen Hülse und Wellenkern erreicht wird. Das Aufschrumpfen der Hülse unterscheidet sich jedoch grundlegend von einer Beschichtung, da bei einer Beschichtung eine festhaftende Schicht aus einem formlosen Stoff aufgetragen wird. Demgegenüber ist die aufgeschrumpfte Hülse nicht festhaftend und hat zudem eine feste Form, weswegen es sich nicht um eine Beschichtung handelt.

Gemäß einer Ausführungsform ist die Hülse über wenigstens einen ersten O-Ring in dem Bohreradapter und/oder über wenigstens einen zweiten O-Ring in dem Antriebsadapter, insbesondere schwimmend, gelagert. Die Hülsenenden der Hülsen werden bei der Montage in den Bohreradapter und den Antriebsadapter eingeschoben. Die O-Ringe sind in den Adaptern angeordnet und fixiert. Durch die O-Ringe wird die Hülse auf diese Weise in den Adaptern gehalten. Ein Innenumfang der O-Ringe entspricht insbesondere einem Außendurchmesser der Hülse. Die Hülsenenden werden bei einer Biegung des Wellenkerns trotz auftretenden Längendifferenzen sicher in den O-Ringen gelagert. Die O-Ringe dichten die Hülse gegen den Wellenkern, den Bohradapter und den Antriebsadapter ab. Insbesondere ist der wenigstens eine O-Ring aus Ethylen-Propylen-Dien-Kautschuk (EPDM) gefertigt.

Insbesondere umfasst der Bohreradapter zwei in Längsrichtung beabstandete erste O-Ringe und/oder der Antriebsadapter zwei in Längsrichtung beabstandete zweite O-Ringe. Durch die O-Ringe und die gasdichten, radialen Verschweißungen ist ein Eindringen von Keimen in das Innere der Markraumbohrerbohrwelle unmöglich.

Bevorzugt weist der Bohreradapter und/oder der Antriebsadapter wenigstens eine nach innen gerichtete, in Umfangsrichtung umlaufende Nut auf, in der der wenigstens eine erste und/oder zweite O-Ring angeordnet ist. Durch die Nuten in den Adaptern werden die O-Ringe in den Adaptern gehalten und fixiert, so dass beim Einschieben der Hülse die O-Ringe auf Position bleiben. Insbesondere sind die ersten O-Ringe statische O-Ringe und die zweiten O-Ringe dynamische O-Ringe. Die Nut bzw. Nuten in dem Antriebsadapter weisen insbesondere eine größere radiale Ausdehnung auf als die Nut bzw. Nuten in dem Bohreradapter. Dadurch wird die Presskraft zwischen den O-Ring bzw. O-Ringen und dem Antriebsadapter gegenüber den Bohreradapter reduziert. Auf diese Weise wird verhindert, dass bei einer Beschädigung des Wellenkerns die auftretenden Torsionskräfte auch die Hülse beschädigen.

Vorzugsweise ist der Bohradapter und/oder der Antriebsadapter an dem Wellenkern verschweißt. Durch eine solche Verschweißung halten die Adapter sicher am Wellenkern. Insbesondere sind der Bohreradapter, der Antriebsadapter und der Wellenkern radial, gasdicht verschweißt. In dieser Ausführungsform bilden der Wellenkern, die Hülse und die Adapter ein Bauteil, dass als Ganzes ausgewechselt und/oder aufbereitet wird.

Bevorzugt weist der Wellenkern einen Durchmesser von 6 mm bis 15 mm auf. Ein solcher Durchmesser des Wellenkerns verschafft der Bohrwelle die notwendige Flexibilität und Stabilität.

Vorzugsweise weist die Bohrung des Schafts einen Durchmesser von 3 mm bis 5,5 mm auf. Durch die Bohrung ist der Schaft kanüliert ausgebildet.

Vorzugsweise ist ein Bohrkopfanschluss unlösbar und gasdicht mit dem Bohreradapter verbunden, wobei der Bohrkopfanschluss dazu ausgebildet ist, lösbar mit einem Bohrkopf verbunden zu werden. Bevorzugt ist ein Antriebsanschluss unlösbar und gasdicht mit dem Antriebsadapter verbunden ist, wobei der Antriebsanschluss dazu ausgebildet ist, lösbar mit einem Bohrerantrieb verbunden zu werden. Insbesondere ist der Bohrkopfanschluss mit dem Bohrkopf verschweißt und/oder der Antriebsanschluss mit dem Antriebsadapter verschweißt. Der Bohrkopfanschluss bildet eine Schnittstelle, an der der Bohrkopf fixiert wird. Je nach Bedarf kann ein unterschiedlicher Bohrkopfanschluss an den Bohreradapter verschweißt werden, um die Bohrkopfwelle mit unterschiedlichen Bohrköpfen oder Bohrköpfen unterschiedlicher Hersteller zu nutzen. Ebenso können verschiedene Antriebsanschlüsse zur Verbindung mit unterschiedlichen Bohrerantrieben angeschweißt werden. Auf diese Weise kann die Markraumbohrerwelle bei der Herstellung an verschiedene Bedürfnisse angepasst werden und dabei stets das gleiche Bauteil bestehend aus Wellenkern, Hülse, Bohreradapter und Antriebsadapter genutzt werden. Dies erleichtert die Herstellung von Markraumbohrern erheblich. Bei dem Bohrerantrieb handelt es sich beispielsweise um eine Antriebsmaschine bzw. eine Bohrmaschine.

Gemäß einer Ausführungsform ist der Bohrkopfanschluss als Mehrkantanschluss für den Bohrkopf und/oder der Antriebsadapter als Mehrkantanschluss zum Anschluss des Antriebsanschlusses und/oder der Antriebsanschluss als Mehrkantanschluss zum Anschluss des Bohrerantriebs ausgebildet. Der Mehrkantanschluss ist beispielsweise als Außensechskantanschluss ausgebildet. Durch einen solchen Anschluss lassen sich die Bauteile zuverlässig, sicher und kraftschlüssig mit der Markraumbohrerbohrwelle verbinden.

Gemäß einer weiteren Ausführungsform weist der Bohreradapter und/oder der Bohreranschluss und/oder der Antriebsadapter und/oder der Antriebsanschluss einen verbreiterten Abschnitt auf, wobei der verbreiterte Abschnitt insbesondere eine umlaufende, rampenförmige Fläche umfasst. Zusammen mit der rampenförmigen Fläche dient dies als Haltefläche für einen Anschluss des Bohrkopfes bzw. des Bohrerantriebs.

Die Aufgabe wird zudem gelöst durch einen Markraumbohrer, umfassend eine Markraumbohrerbohrwelle nach einer der zuvor diskutierten Ausführungsformen und einen Bohrkopf, wobei der Bohrkopf dazu ausgebildet ist, lösbar mit der Markraumbohrerbohrwelle verbunden zu werden.

Der Markraumbohrer verkörpert die gleichen Vorteile, Merkmale und Eigenschaften wie die zuvor beschriebene Bohrwelle.

Vorzugsweise weist der Bohrkopf einen Bohrwellenanschluss auf, der dazu ausgebildet ist, insbesondere formschlüssig, mit dem Bohrkopfanschluss verbunden zu werden.

Vorzugsweise umfasst der Markraumbohrer einen Bohrerantrieb, wobei der Bohrerantrieb einen Bohrwellenanschluss aufweist, der dazu ausgebildet ist, insbesondere formschlüssig, mit dem Antriebsanschluss verbunden zu werden.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Im Rahmen der Erfindung sind Merkmale, die mit "insbesondere" oder "vorzugsweise" gekennzeichnet sind, als fakultative Merkmale zu verstehen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: eine schematisch vereinfachte perspektivische Explosionsdarstellung einer Ausführungsform eines Markraumbohrers,
- Fig. 2: eine schematisch vereinfachte Ansicht des Markraumbohrers aus Fig. 1 im zusammengesetzten Zustand,
- Fig. 3: eine schematisch vereinfachte perspektivische Darstellung einer Markraumbohrerbohrwelle mit einem Bohrkopfanschluss,
- Fig. 4: eine Querschnittsdarstellung der Markraumbohrerbohrwelle aus Fig. 3,
- Fig. 5A, 5B: vergrößerte Darstellungen der Ausschnitte A und B aus Fig. 4,
- Fig. 5C, 5D: Querschnittsansichten entlang der Schnittlinien C-C und D-D aus Fig. 4,
- Fig. 6: eine schematisch vereinfachte perspektivische Darstellung einer Markraumbohrerbohrwelle mit einem Bohrkopfanschluss und einem Antriebsanschluss,
- Fig. 7: eine schematisch vereinfachte Seitenansicht der Markraumbohrerbohrwelle aus Fig. 6, und
- Fig. 8: eine schematisch vereinfachte Querschnittsansicht durch die Schnittebene A-A aus Fig. 7.

In den Zeichnungen sind jeweils gleiche oder gleichartige Elemente und/oder Teile mit denselben Bezugsziffern versehen, so dass von einer erneuten Vorstellung jeweils abgesehen wird.

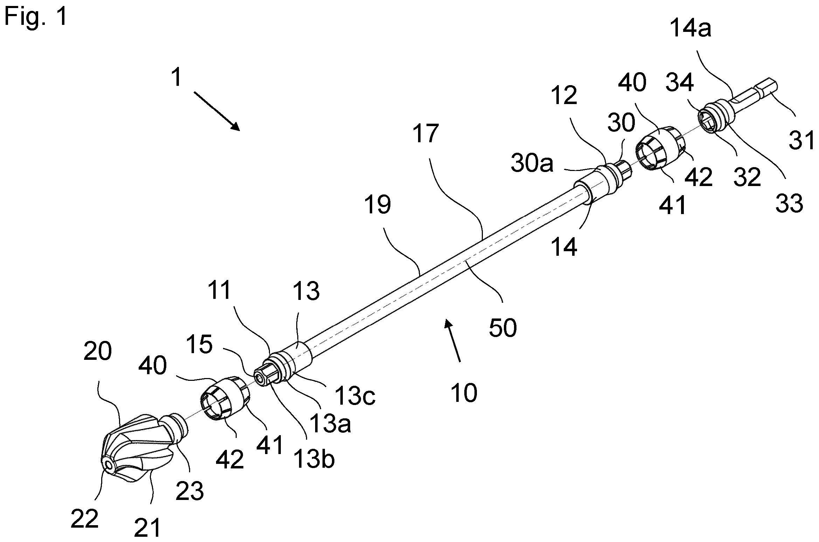

Fig. 1 zeigt schematisch vereinfacht eine erste Ausführungsform eines Markraumbohrers 1 in einer perspektivischen Explosionsansicht. Der Markraumbohrer 1 umfasst eine Markraumbohrerbohrwelle 10, einen Bohrkopf 20, einen Antriebsanschluss 14a und zwei Verrieglungskörper 40. Der Bohrkopf 20 weist eine Reihe Schneiden 21 auf, um die Markhöhle im Knochen eines Patienten aufzubohren. Zu diesem Zweck umfasst der Bohrkopf 20 auch eine längsaxiale Bohrung 22, die sich vollständig durch den Bohrkopf 20 erstreckt. Hinter den Schneiden 21 weist der Bohrkopf 20 einen hohlzylinderförmigen Abschnitt auf, auf dem eine umlaufende rampenförmige Fläche 23 ausgebildet ist, die zur Fixierung des Bohrkopfs 20 an der Markraumbohrerbohrwelle 10 vorgesehen ist. Dieser Abschnitt ist somit als Schaftanschluss für die Markraumbohrerbohrwelle 10 ausgebildet.

Die Markraumbohrerbohrwelle 10 ist flexibel ausgebildet und dazu ausgestaltet, die Drehmomente aufzunehmen, die am Markraumbohrer 1 anliegen. Dazu ist ein Wellenkern 18 des Schafts 19 der Markraumbohrerbohrwelle 10 aus Nitinol gefertigt, welches der Markraumbohrerbohrwelle 10 die nötige Flexibilität und Stabilität verleiht. Um im Falle eines Bruchs des Wellenkerns 18 ein Eindringen von Nitinolsplittern in den Patientenkörper zu verhindern, umschließt eine Hülse 17 eine Mantelfläche 16 des Wellenkerns 18. Die Hülse 17 besteht aus einem Kunststoff, beispielsweise Polyetheretherketon (PEEK). Zum Anschluss des Bohrkopfs 20 und des Anschlussadapters 30 ist an einem ersten Ende 11 des Wellenkerns 18 bzw. des Schafts 19 ein Bohreradapter 13 angeschweißt und an einem gegenüberliegenden zweiten Ende 12 ein Antriebsadapter 14.

An dem Bohreradapter 13 ist ein Bohrkopfanschluss 13a verschweißt, der zur Fixierung des Bohrkopfs 20 ausgebildet ist. Der Bohrkopfanschluss 13a weist einen Mehrkantanschluss 13b auf, der in eine aus perspektivischen Gründen nicht sichtbare Mehrkantaufnahme im Bohrkopf 20 greift. Der Bohreradapter 13 oder der Bohrkopfanschluss 13a umfasst zudem einen verbreiterten Abschnitt und weist eine umlaufende, rampenförmige Fläche 13c auf. Zur Fixierung des Bohrkopfs 20 an der Markraumbohrerbohrwelle 10 ist ein Verriegelungskörper 40 vorgesehen, der hülsenförmig ausgebildet ist und an beiden Enden jeweils ein federndes, nach innen vorstehendes Halteelement 41, 42 aufweist. Bei diesem Halteelement 41, 42 handelt es sich beispielsweise um einen Haltekranz. Das Halteelement 41, 42 greift hinter die rampenförmigen Flächen 13c, 23 und hält so den Bohrkopf 20 an der Markraumbohrerbohrwelle 10.

Der Antriebsadapter 14 weist ebenfalls einen Mehrkantanschluss 30 und eine rampenförmige Fläche 30a auf. Ebenso weist der Antriebsanschluss 14a eine mehrkantförmige Innenbohrung 34 auf, die formschlüssig zum Mehrkantanschluss 30 der Markraumbohrerbohrwelle 10 ausgebildet ist. Die Außenfläche des Antriebsanschlusses 14a umfasst zudem eine rampenförmige Fläche 33, die wie zuvor beschrieben eine Haltefläche bildet und mit einem Halteelement 42 eines weiteren Verriegelungskörpers 40 wechselwirkt, um den Antriebsanschluss 14a an der Markraumbohrerbohrwelle 10 zu fixieren. Genau wie der Bohrkopf 20 weist auch die Markraumbohrerbohrwelle 10 eine längsaxiale Bohrung 15 und der Antriebsanschluss 14a eine längsaxiale Bohrung 32 auf, die sich jeweils vollständig in Richtung einer Längsachse 50 durch die Bauteile erstrecken. Der Markraumbohrer 1 ist somit kanüliert. Zur Aufnahme des Drehmoments von einer nicht gezeigten Antriebsmaschine bzw. Bohrmaschine ist das hintere Ende des Antriebsanschlusses 14a als Maschinenanschluss 31 ausgeformt.

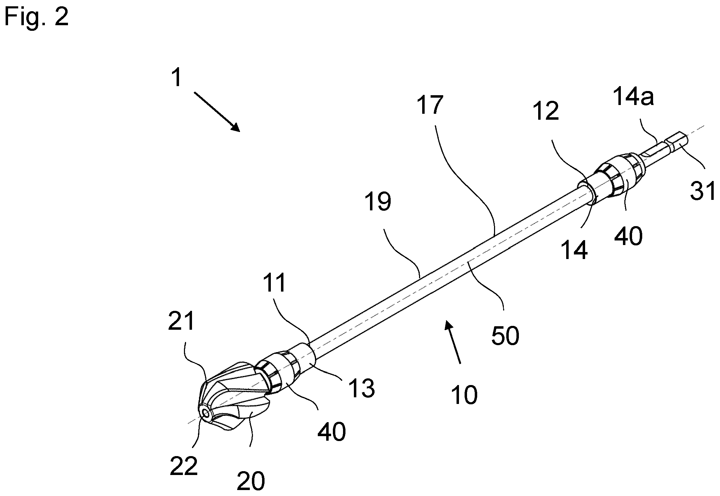

Fig. 2 zeigt den Markraumbohrer 1 im zusammengesetzten Zustand. In diesem Zustand sind der Bohrkopf 20 und der Antriebsanschluss 14a mittels der Verriegelungskörper 40 an der Markraumbohrerbohrwelle 10 fixiert. Auf diese Weise umfasst der resultierende Markraumbohrer 1 mehrere separat wiederaufbereitbare Einzelteile, nämlich die Markraumbohrerbohrwelle 10, den Bohrkopf 20, den Antriebsanschluss 14a und die Verriegelungskörper 40.

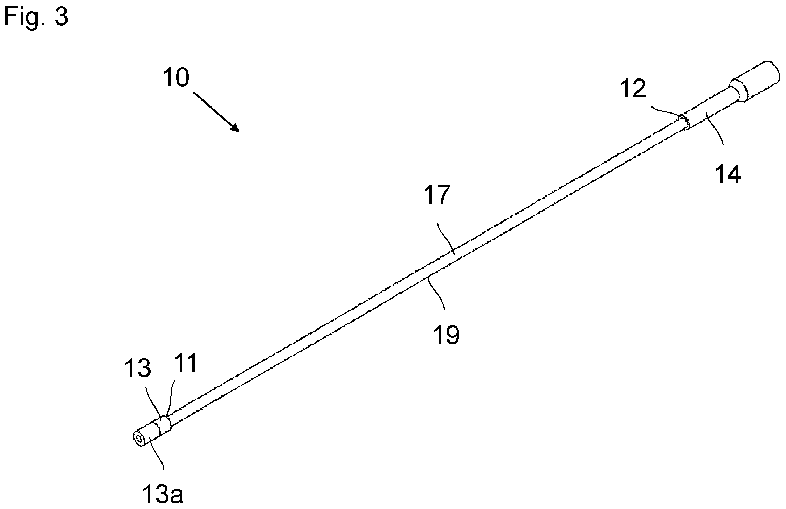

Fig. 3 zeigt schematisch vereinfacht eine weitere Ausführungsform einer Markraumbohrerbohrwelle 10 in perspektivischer Darstellung. Auch bei dieser Ausführungsform umfasst der Schaft 19 einen Wellenkern 18 aus Nitinol, der in Fig. 3 nicht sichtbar ist, da er vollständig von der Hülse 17 aus Kunststoff umhüllt ist. Am ersten Ende 11 des Schafts 19 ist der Bohreradapter 13 an dem Wellenkern 18 fixiert, beispielsweise festgeschweißt. In Fig. 3 ist der Wellenkern 18 nicht sichtbar, da er von der Hülle 17 verdeckt ist. Der Bohrkopfanschluss 13a ist an dem Bohreradapter 13 verschweißt. Ebenso ist am zweiten Ende 12 des Schafts 19 der Antriebsadapter 14 fixiert, z.B. festgeschweißt. In dieser Ausführungsform ist der Bohreradapter 13 im Wesentlichen zylinderförmig ausgebildet. Der Antriebsadapter 14 hat einen zylinderförmigen Bereich, an den ein Kegelstumpf anschließt, auf den wiederum ein zylinderförmiger Bereich mit größerem Radius folgt. Ein Antriebsanschluss 14a ist in dieser Ausführungsform noch nicht an dem Antriebsadapter 14 fixiert bzw. verschweißt und daher nicht dargestellt.

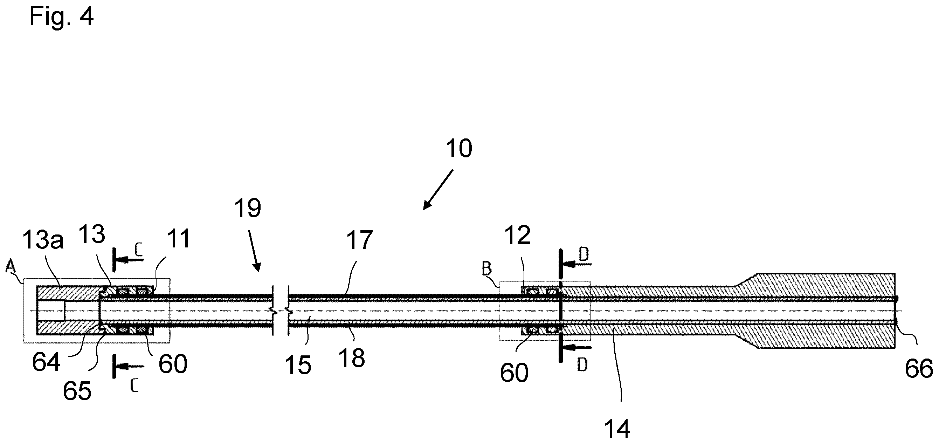

In Fig. 4 ist eine Querschnittsdarstellung der Markraumbohrerbohrwelle 10 aus Fig. 3 gezeigt. In dieser Darstellung ist die Bohrung 15 gut sichtbar, die sich durch die gesamte Markraumbohrerbohrwelle 10 erstreckt. Zudem ist zu erkennen, wie der Bohreradapter 13 mit einem umlaufenden Schweißring 64 an dem Wellenkern 18 fixiert ist. Dazu ist der äußerte Bereich des Wellenkerns 18, der weit innerhalb des Bohreradapters 13 angeordnet ist, nicht mit der Hülse 17 bedeckt, um eine Verschweißung der Bauteile zu erleichtern. Ein weiterer Schweißring 65 verbindet den Bohreradapter 13 mit dem Bohrkopfanschluss 13a. Schließlich ist ein Schweißring 66 am äußeren Ende des Antriebsadapters 14 vorgesehen, um dort den Antriebsadapter 14 an dem Wellenkern 18 zu fixieren.

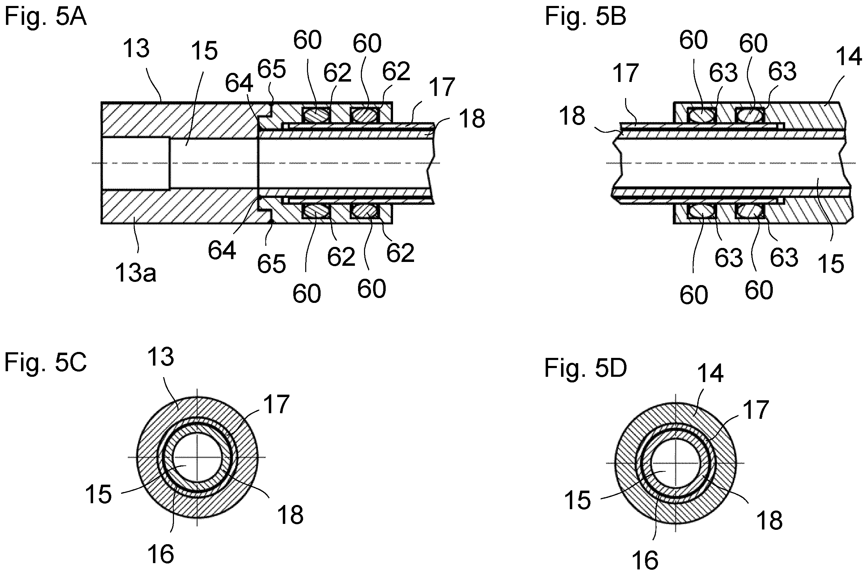

Um die Hülse 17 zu lagern, umfassen die Adapter 13, 14 jeweils zwei O-Ringe 60. In den vergrößerten Abschnitten A und B, die in den Fig. 5A und 5B dargestellt sind, ist dies noch deutlicher zu erkennen. Es wird ersichtlich, dass die O-Ringe 60 in dem Bohreradapter 13 in umlaufenden, nach innen gerichteten Nuten 62 angeordnet sind. In dem Antriebsadapter 14 sind die O-Ringe 60 in umlaufenden, nach innen gerichteten Nuten 63 angeordnet. Die Nuten 63 haben in radialer Richtung eine etwas größere Ausdehnung als die Nuten 62, so dass in dem Antriebsadapter 14 die O-Ringe 60 eine geringere Presskraft ausüben als in dem Bohreradapter 13. dadurch sind die O-Ringe 60 in dem Bohreradapter 13 als statische O-Ringe 60 und die O-Ringe 60 in dem Antriebsadapter 14 als dynamische O-Ringe 60 ausgebildet. Bei einem Bruch des Wellenkerns 18 durch zu hohe Torsionskräfte wird durch die dynamischen O-Ringe 60 ein Bruch der Hülse 17 verhindert. Bei der Montage der Markraumbohrerbohrwelle 10 wird die Hülse 17 in die Adapter 13, 14 eingeschoben und/oder es werden die Adapter 13, 14 über die Hülse 17 gestülpt, so dass die Hülse 17 in den O-Ringen 60 schwimmend gelagert ist. Beispielsweise wird zuerst der Wellenkern 18 mit dem Bohreradapter 13 mittels des Schweißrings 64 verschweißt, bevor der Bohreranschluss 13a mittels des Schweißrings 65 am Bohreradapter 13 fixiert wird. Anschließend wird die Hülse 17 in den Bohreradapter 13 eingeschoben. Schließlich wird der Antriebsadapter 14 über den Wellenkern 18 und die Hülse 17 geschoben, bevor mit dem Schweißring 66 der Wellenkern 18 dauerhaft an dem Antriebsadapter 14 fixiert wird.

Die Fig. 5C und 5D zeigen Querschnitte entlang der Schnittlinien C-C und D-D aus Fig. 4. Diese Querschnitte verdeutlichen den Aufbau der Markraumbohrerbohrwelle 10. An den Enden 11, 12 umschließen die Adapter 13, 14 die Hülse 17, welche um die Mantelfläche 16 des Wellenkerns 18 geschoben ist. Ein Abstand zwischen der Innenseite der Hülse 17 und der Mantelfläche 16 des Wellenkerns 18 beträgt im Mittel etwa 0,01 mm bis 0,02 mm. Dieses Spaltmaß erhöht die impulsverringernden Eigenschaften der Hülse 17 im Gegensatz zu einer direkt aufgebrachten Beschichtung weiter. Da das Spaltmaß jedoch sehr gering ist, ist dies in den Fig. 5C und 5D nicht zu erkennen. Der Wellenkern 18 weist in der Mitte die Bohrung 15 auf. Dies ist bei allen Markraumbohrerbohrwellen 10 der Fall und unterscheidet diese von vielen anderen Arten von Bohrwellen. Durch die Bohrung 15 wird die Stabilität im Vergleich zu einer Bohrwelle ohne Bohrung verringert, wodurch die impulsverringernde Hülse 17 notwendig wird.

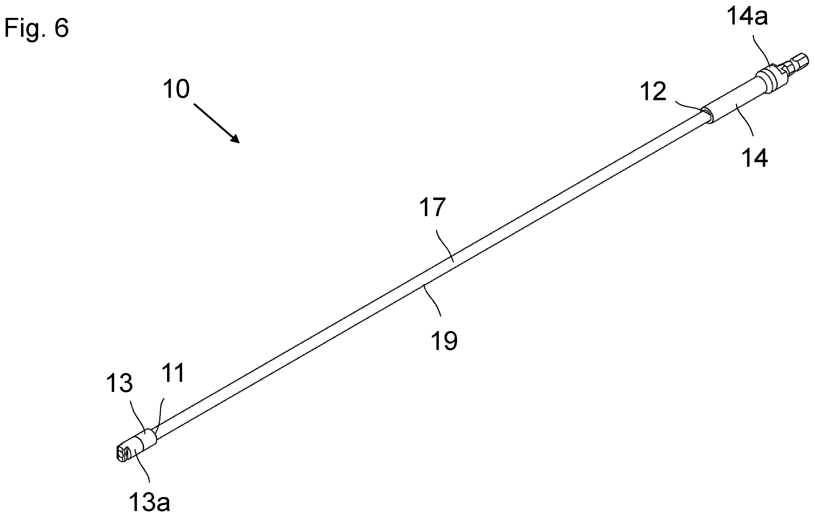

Fig. 6 zeigt schematisch vereinfacht eine weitere Ausführungsform einer Markraumbohrerbohrwelle 10 in perspektivischer Darstellung. Im Gegensatz zu der Ausführungsform aus Fig. 3 ist an dem Antriebsadapter 14 ein Antriebsanschluss 14a angeschweißt. Zudem unterscheidet sich die Form des Bohrkopfanschlusses 13a im Vergleich zu der Ausführungsform aus Fig. 3. Außer den Anschlüssen 13a, 14a ist die Ausführungsform jedoch im Wesentlichen unverändert, mit Ausnahme geringfügiger Unterschiede in der Form des Antriebsadapters 14. Auf diese Weise kann das Bauteil bestehend aus Wellenkern 18, Hülse 17, Bohreradapter 13 und Antriebsadapter 14 immer in der gleichen Art hergestellt werden. Um den Markraumbohrerbohrwelle 10 an unterschiedliche Bohrköpfe 20 und/oder Bohrerantriebe 30 anzupassen müssen lediglich andere Anschlüsse 13a, 14a angeschweißt werden.

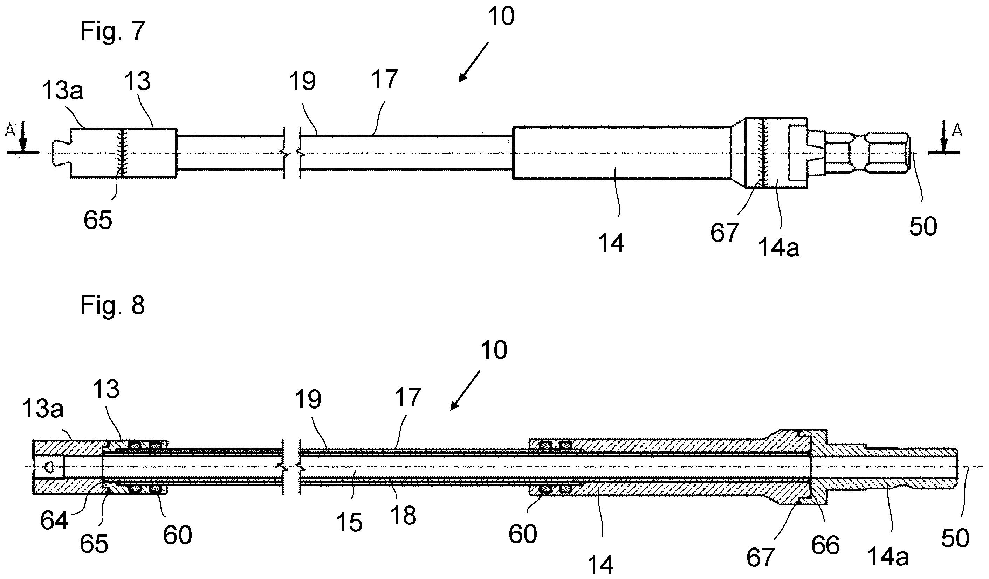

Fig. 7 zeigt die Markraumbohrerbohrwelle 10 in einer Seitenansicht. In dieser Ansicht ist der Schweißring 65 zur Verbindung des Bohreradapters 13 und des Bohrkopfanschlusses 13a sowie die Schweißring 67 zur Verbindung des Antriebsadapters 14 mit dem Antriebsanschluss 14a gut zu erkennen. In der Querschnittsansicht in Fig. 8, die sich entlang der A-A Schnittlinie erstreckt, sind zudem die Schweißringe 64, 66 sichtbar, welche den Wellenkern 18 mit den Adaptern 13, 14 verbinden. Im Übrigen gleicht die Ausführungsform der Markraumbohrerbohrwelle 10 aus Fig. 4.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein.

### Bezugszeichenliste

- 1: Markraumbohrer
- 10: Markraumbohrerbohrwelle
- 11: erstes Ende
- 12: zweites Ende
- 13: Bohreradapter
- 13a: Bohrkopfanschluss
- 13b: Mehrkantanschluss
- 13c: rampenförmige Fläche
- 14: Antriebsadapter
- 14a: Antriebsanschluss
- 15: Bohrung
- 16: Mantelfläche
- 17: Hülse
- 18: Wellenkern
- 19: Schaft
- 20: Bohrkopf
- 21: Schneide
- 22: Bohrung
- 23: rampenförmige Fläche
- 30: Mehrkantanschluss
- 30a: rampenförmige Fläche
- 31: Maschinenanschluss
- 32: Bohrung
- 33: rampenförmige Fläche
- 34: mehrkantförmige Innenbohrung
- 40: Verriegelungskörper
- 41: Halteelement
- 42: Halteelement
- 50: Längsachse
- 60: O-Ring
- 62: Nut
- 63: Nut
- 64: Schweißring
- 65: Schweißring
- 66: Schweißring
- 67: Schweißring

## Patentansprüche

1. Markraumbohrerbohrwelle (10), umfassend einen längserstreckten Schaft (19), wobei der Schaft (19) einen Wellenkern (18) mit einer durchgehenden, längsaxialen Bohrung (15) aufweist, wobei der Wellenkern (18) aus Nitinol gefertigt ist, wobei an einem ersten Ende (11) des Schafts (19) ein Bohreradapter (13) unlösbar mit dem Wellenkern (18) verbunden ist, wobei an einem gegenüberliegenden zweiten Ende (12) ein Antriebsadapter (14) unlösbar mit dem Wellenkern (18) verbunden ist, wobei eine Mantelfläche (16) des Wellenkerns (18) von einer impulsverringernden Hülse (17) aus Kunststoff umschlossen ist, wobei sich die Hülse (17) von dem ersten Ende (11) durchgehend bis zu dem zweiten Ende (12) erstreckt.

2. Markraumbohrerbohrwelle (10) nach Anspruch 1, **dadurch gekennzeichnet, dass** die Mantelfläche (16) von dem Bohreradapter (13) bis zum Antriebsadapter (14) durchgehend von der Hülse (17) ummantelt ist.

3. Markraumbohrerbohrwelle (10) nach Anspruch 1 oder 2, **dadurch gekennzeichnet, dass** die Hülse (17) eine Wandungsstärke von wenigstens 0,3 mm, insbesondere wenigstens 0,5 mm, aufweist.

4. Markraumbohrerbohrwelle (10) nach einem der Ansprüche 1 bis 3, **dadurch gekennzeichnet, dass** die Hülse (17) ein Spaltmaß von wenigstens 0,01 mm zur Mantelfläche (16) des Wellenkerns (18) aufweist.

5. Markraumbohrerbohrwelle (10) nach einem der Ansprüche 1 bis 3, **dadurch gekennzeichnet, dass** die Hülse (17) auf den Wellenkern aufgeschrumpft ist, so dass die Hülse (17) insbesondere ein Spaltmaß von weniger als 0,01 mm zur Mantelfläche (16) des Wellenkerns (18) aufweist.

6. Markraumbohrerbohrwelle (10) nach einem der Ansprüche 1 bis 5, **dadurch gekennzeichnet, dass** die Hülse (17) über wenigstens einen ersten O-Ring (60) in dem Bohreradapter (13) und/oder über wenigstens einen zweiten O-Ring (60) in dem Antriebsadapter (14), insbesondere schwimmend, gelagert ist.

7. Markraumbohrerbohrwelle (10) nach Anspruch 6, **dadurch gekennzeichnet, dass** der Bohreradapter (13) und/oder der Antriebsadapter (14) wenigstens eine nach innen gerichtete, in Umfangsrichtung umlaufende Nut (62, 63) aufweist, in der der wenigstens eine erste und/oder zweite O-Ring (60) angeordnet ist.

8. Markraumbohrerbohrwelle (10) nach einem der Ansprüche 1 bis 7, **dadurch gekennzeichnet, dass** der Bohradapter (13) und/oder der Antriebsadapter (14) an dem Wellenkern (18) verschweißt ist.

9. Markraumbohrerbohrwelle (10) nach einem der Ansprüche 1 bis 8, **dadurch gekennzeichnet, dass** der Wellenkern (18) einen Durchmesser von 6 mm bis 15 mm aufweist.

10. Markraumbohrerbohrwelle (10) nach einem der Ansprüche 1 bis 9, **dadurch gekennzeichnet, dass** die Bohrung (15) des Schafts (19) einen Durchmesser von 3 mm bis 5,5 mm aufweist.

11. Markraumbohrwelle (10) nach einem der Ansprüche 1 bis 10, **dadurch gekennzeichnet, dass** ein Bohrkopfanschluss (13a) unlösbar und gasdicht mit dem Bohreradapter (13) verbunden ist, wobei der Bohrkopfanschluss (13a) dazu ausgebildet ist, lösbar mit einem Bohrkopf (20) verbunden zu werden.

12. Markraumbohrwelle (10) nach einem der Ansprüche 1 bis 11, **dadurch gekennzeichnet, dass** ein Antriebsanschluss (14a) unlösbar und gasdicht mit dem Antriebsadapter (14) verbunden ist, wobei der Antriebsanschluss (14a) dazu ausgebildet ist, lösbar mit einem Bohrerantrieb (30) verbunden zu werden.

13. Markraumbohrer (1), umfassend eine Markraumbohrerbohrwelle (10) nach einem der Ansprüche 1 bis 12 und einen Bohrkopf (20), wobei der Bohrkopf (20) dazu ausgebildet ist, lösbar mit der Markraumbohrerbohrwelle (10) verbunden zu werden.
